**Europäisches Patentamt**

**European Patent Office**

**Office européen des brevets**

(19)

(11) Veröffentlichungsnummer: **O 001 083**

**B1**

(12) # EUROPÄISCHE PATENTSCHRIFT

(21) Anmeldenummer: **78100767.9**

(22) Anmeldetag: **28.08.78**

(51) Int. Cl.³: **C 07 C 69/96,**
**C 07 C 68/06,**
**B 01 J 23/08**

(54) **Verfahren zur Herstellung von Dialkylcarbonaten**

(30) Priorität: **07.09.77 DE 2740251**

(43) Veröffentlichungstag der Anmeldung:
**21.03.79 Patentblatt 79/06**

(45) Bekanntmachung des Hinweises auf die Patenterteilung:
**03.09.80 Patentblatt 80/18**

(84) Benannte Vertragsstaaten:
**BE CH DE FR GB LU NL**

(56) Entgegenhaltungen:
**FR - A - 2 276 284**

(73) Patentinhaber: **Bayer AG**
**Zentralbereich Patente, Marken und Lizenzen**
**D - 5090 Leverkusen 1, Bayerwerk (DE)**

(72) Erfinder: **Krimm, Heinrich, Dr.**
**Heyenbaumstrasse 65**
**D - 4150 Krefeld (DE)**
**Buysch, Hans-Josef, Dr.**
**Brandenburgerstrasse 28**
**D - 4150 Krefeld (DE)**
**Rudolph, Hans, Dr.**
**Haydnstrasse 9**
**D - 4150 Krefeld (DE)**

## Verfahren zur Herstellung von Dialkylcarbonaten

Die vorliegende Erfindung betrifft ein Verfahren zur Herstellung von Dialkylcarbonaten durch Umsetzung von Glykolcarbonaten mit Alkoholen in Gegenwart von Thalliumverbindungen.

Aus der US—PS 3 642 858 ist die Herstellung von Dialkylcarbonaten durch Umesterung von Alkylcarbonaten mit Alkoholen in Gegenwart von Alkalimetallen oder Alkalimetallverbindungen bekannt. Die bevorzugte Verwendung von Natriumalkoholaten als Katalysator legt deren besondere Eignung nahe. Als typische Reaktionstemperaturen sind solche zwischen 175 und 225°C angegeben. Neidrigere Temperaturen führen gemäß Beispiel 3 der Tabelle der US—PS auch bei längeren Reaktionszeiten nur zu geringen Umsätzen. Werden jedoch die bevorzugten hohen Reaktionstemperaturen angewandt, so finden zum Teil in beträchtlichem Maße Nebenreaktionen statt, die die Ausbete mindern und die Aufarbeitung erschweren. Insbesondere finden solche Nebenreaktionen bei den aliphatischen Carbonaten, die weniger stabil sind, statt. Diese spalten vor allem leicht Kohlendioxid ab unter Bildung von Äthern. Solche Nebenreaktionen finden bevorzugt zwischen Dialkylcarbonaten und 1,2-Glykolen statt, besonders leicht zwischen Glykolcarbonaten und 1,2-Glykolen, wenn im Verlauf der Umesterung der Glykolanteil zunimmt. Die sich bildenden Nebenprodukte sind vor allem Alkylglykoläther und Polyglykole, wie Di-, Tri- und Tetraglykole.

Weiterhin ist in der DT—OS 2 615 665 ein Verfahren zur Herstellung von Dialkylcarbonaten durch Umesterung von cyclischen Carbonaten mit Alkoholen in Gegenwart von tertiären Aminen bei Temperaturen zwischen 50 und 150°C beschrieben. Nachteilig bei diesem Verfahren ist die große Menge an tertiären Aminen, die als Katalysator benötigt werden. Da die tertiären Amine selbst flüchtig sind, sind sie schwer von den Reaktionskomponenten abzutrennen. Trotz der großen Katalysatormengen verläuft die Umesterung gemäß dem Verfahren der DT—OS 2 615 665 relativ langsam. Zur Beschleunigung müßte demnach die Temperatur angehoben werden. Eigene Versuche haben jedoch gezeigt, daß dann zwar die Reaktionsgeschwindigkeit zunimmt, gleichzeitig aber vermehrt Nebenreaktionen im oben beschriebenen Sinne stattfinden.

Es wurde nun überraschend gefunden, daß man die Umsetzung von Glykolcarbonaten mit Alkoholen zu Dialkylcarbonaten unter weit milderen Bedingungen und in sehr viel kürzerer Zeit als aus dem Stand der Technik bekannt ist, durchführen kann, wenn man Thallium-Verbindungen als Katalysatoren verwendet. Die Thallium-Verbindungen besitzen im Hinblick auf diese Reaktion einen außerordentlich günstigen Temperatur-Koeffizienten, der bei nur geringfügiger Temperaturerhöhung einen sehr raschen Geschwindigkeitsanstieg bewirkt.

Dies ist insofern überraschend, als Thallium-Verbindungen zwar als Umesterungskatalysatoren bekannt sind (US—PS 3 506 619, Beispiel V; DE—OS 26 37 409), jedoch im Zusammenhang mit Carbonaten nicht beschrieben wurden. Überraschend ist ferner auch, daß Thallium-Verbindungen die Nebenreaktionen gegenüber anderen Umesterungskatalysatoren unter sonst gleichen Bedingungen zurückdrängen.

Gegenstand der vorliegenden Erfindung ist ein Verfahren zur Herstellung von Dialkylcarbonaten durch Umsetzung von Glykolcarbonaten mit Alkoholen bei erhöhter Temperatur in Gegenwart von Katalysatoren, das dadurch gekennzeichnet ist, daß man die Reaktion in Gegenwart von Thallium-Verbindungen als Katalysatoren bei Temperaturen im Bereich von 50 bis 250°C durchführt.

Das erfindungsgemäße Verfahren besitzt gegenüber dem Stand der Technik folgende Vorteile:

Die Katalysator-Mengen sind gering. Sie betragen nur einen Bruchteil der üblicherweise verwendeten Menge, was bedeutet, daß der Katalysator ohne Schwierigkeit vom Reaktionsgemisch abgetrennt und wieder verwendet werden kann. Die durch hohe Katalysatorkonzentrationen verursachten Zersetzungen bei der destillativen Aufarbeitung der Produkte werden vermieden. Die Reaktionstemperaturen können vergleichsweise niedrig gehalten werden, was zur Erhöhung der Wirtschaftlichkeit des Verfahrens beiträgt. Die Reaktionsgeschwindigkeit ist außerordentlich groß und gestattet es, daß sich in kürzester Zeit das Umesterungsgleichgewicht einstellt, was eine hohe Raum/Zeit-Ausbeute gewährleistet. Nebenreaktionen, wie die Bildung von Polyglykolen werden praktisch vollkommen unterdrückt und damit Ausbeuteverluste vermieden.

Als Ausgangsprodukte für das erfindungsgemäße Verfahren werden einersetis aliphatische- und/oder cycloaliphatische Hydroxyverbindungen mit 1 bis 10 Kohlenstoffatomen wie Methanol, Äthanol, Propanol, Isopropanol, N-Butanol, Isobutanol, Allylalkohol, Cyclohexanol, Äthylhexanol, Benzylalkohol, Methylglykol andererseits Carbonate von 1,2-Diolen mit 2 bis 4 Kohlenstoffatomen, wie Butylencarbonat, Vinyl-Äthylenglykolcarbonat, Chlormethl-Äthylenglykolcarbonat, besonders bevorzugt Äthylenglykolcarbonat und Propylenglykolcarbonat eingesetzt.

Bevorzugt werden als aliphatische und/oder cycloaliphatische Hydroxyverbindungen solche mit 1 bis 6 C-Atomen eingesetzt, wobei solche mit 1 bis 4 C-Atomen besonders bevorzugt sind.

Das Molverhältnis der Reaktionspartner ist nicht sehr entscheidend. Alkohol kann sowohl im Unter- wie im Überschuß verwendet werden. Ein Überschuß an Alkohol von etwa 1 bis 10 Mol pro Mol Glykolcarbonat empfiehlt sich jedoch, um das Gleichgewicht in Richtung des gewünschten Carbonats zu verschieben. Natürlich kann auch ein größerer Überschuß eingesetzt werden. Geeignete Katalysatoren sind Verbindungen des Thalliums wie Thallium-I-oxid, Thallium-III-oxid, Thallium-I-hydroxid, Thallium - I - carbonat, Thallium - I - acetat, Thallium-III-acetat, Thallium-I-bromid, Thallium-I-chlorid, Thallium-III-chlorid, Thallium-I-fluorid, Thallium - I - formiat, Thallium - I - nitrat, Thallium-I-cyanat, Thallium-I-stearat. Thallium-I-naphthenat, Thalliumbenzoat, Thalliumcyclohexylphosphonat, Thallium-I-hexahydrobenzoat, Cyclopentadienylthallium, Thalliummethylat, Thalliumäthylat.

Bevorzugt werden folgende Thalliumverbindungen eingesetzt: Thallium-I-oxid, Thallium-I- hydroxid, Thallium - I - carbonat, Thallium - I - acetat, Thallium-III-acetat, Thallium-I-fluorid, Thallium-I-formiat, Thallium-I-nitrat, Thallium-I-naphthenat, Thalliummethylat.

Die Mengen an Katalysator sind richt kritisch. Sie betragen im allgemeinen etwa 10 bis 0,0001 Gew.-%, vorzugsweise 0,001 bis 1 Gew.-%, bezogen auf das Reaktionsgemisch.

Die Reaktionstemperaturen liegen im Bereich von etwa 50 bis 250°, vorzugsweise bei 60 bis 220°C, besonders bevorzugt bei 120 bis 200°C.

Die notwendige Verweilzeit im Reaktor ist temperaturabhängig. Sie leigt zwischen gut 10 Standen bei neidriger Temperatur und wenigen Sekunden bei höheren Temperaturen, vorzugsweise im Bereich von 0,1 bis 20 Minuten. Sie kann aber ohne Gefahr der Nebenproduktbildung verlängert werden.

Das erfindungsgemäße Verfahren wird vorzugsweise nach 3 Varianten durchgeführt.

Die erste ist allerdings auf Methanol beschränkt. Sie besteht darin, daß man aus einem auf Siedetemperatur erhitzten Gemisch von Methanol, Alkylencarbonat und Katalysator das Methanol/Dimethylcarbonat-Azeotrop abdestilliert.

Nach der zweiten leitet man durch, auf über den Siedepunkt des Alkohols erhitztes, Alkylencarbonat Alkoholdampf hindurch und kondensiert in einer nachgeschalteten Vorlage eine Mischung aus Alkohol und Dialkylcarbonat.

Die dritte Methode schließlich beinhaltet das Erhitzen von Alkohol, Alkylencarbonat und Katalysator auf Temperaturen oberhalb 100°, vorzugsweise 120° bis 200°C, und nachfolgende destillative Auftrennung des Reaktionsgemisches.

Die Reaktion kann drucklos durchgeführt werden. Bei niedrig siedenden Komponenten ist allerdings ein Druckreaktor erforderlich, wenn die Reaktion im oberen Temperaturbereich durchgeführt werden soll. Der Druck ist nicht kritisch. Im allgemeinen lässt man die Reaktion unter dem Eigendruck der Reaktanten ablaufen. Man kann allerdings auch unter erhöhtem Druck, z.B. unter einer Schutzgasatmosphäre arbeiten. Dabei ist ein Druck von etwa 2 bis 100 bar zweckmäßig.

Die Produkte des erfindungsgemäßen Verfahrens sind geeignet als Lösungsmittel für Cellulosederivate und also Ausgangsprodukte für die Herstellung von Diarylcarbonaten, aliphatischen und aromatischen Polycarbonaten, Arznei- und Pflanzenschutzmittel (vgl. z.B. DT—OS 2 528 412, DT—AS 1 031 512, Amer. Chem. Soc. 52, 314 (1930), Ullmanns Encyklopädie der technischen Chemie 3. Aufl., Bd. 9, S. 776 ff.

Das erfindungsgemäße Verfahren läßt sich anhand der folgenden Beispiele räher erläutern, ohne es jedoch auf diese Beispiele einzuschränken.

### Beispiel 1

a) Ein Gemisch von 370 g (4,2 Mol) Äthylenglykolcarbonat, 672 g (21 Mol) Methanol und 0,2 g Thalliumcarbonat wird im Autoklaven unter Rühren und Stickstoff in ca. 30' auf 150°C gebracht. Bei Erreichen dieser Temperatur wird eine Probe genommen und gaschromatographisch analysiert. Sie enthält 25 Gew.-% Dimethylcarbonat. Durch Verlängerung der Reaktionszeit bei 150°C ändert sich dieser Gehalt laut Gaschromatographie nicht mehr. Nach 2 h bei 150°C wird abgekühlt und der Autoklavinhalt destilliert. Dabei werden Methanol und Dimethylcarbonat sehr rasch bei Normaldruck bzw. etwas reduziertem Druck vom Katalysator abdestilliert, um das Verhältnis der Reaktionskomponenten nicht durch weitere Umesterung zu verschieben und in Kühlfallen kondensiert. Man erhält 731g Destillat mit einem Gehalt an Dimethylcarbonat von 36,0 Gew.-% d.s. 263g, entsprechend einem Umsatz an Äthylenglykolcarbonat zu Dimethylcarbonat von 70%.

Danach wird im Ölvakuum der höhersiedende, aus Äthylenglykol und Äthylenglykolcarbonat bestehende Anteil des Reaktionsgemisches vom Katalysator rasch bis zu einer Sumpftemperatur von 113°C und einer Kopftemperatur von 63—75°/133—200 Pa abdestilliert. Man erhält 294g Destillat, das zu 62 Gew.-% aus Äthylenglykol und 38 Gew.-% aus Äthylenglykolcarbonat besteht. Als Destillationsrückstand verbleiben 0,3g. Das entspricht praktisch der eingesetzten Katalysatormenge.

Aufarbeitungsverlust ca. 1,5 Gew.-%, bezogen auf das gesamte Reaktionsgemisch. Die Ausbeute an Dimethylcarbonat bez. auf umgesetztes Äthylenglykolcarbonat beträgt 98—99% d.Th.

b) Das aus Beispiel 1a erhaltene Gemisch (294g) aus Äthylenglykol (62%) und Äthylenglykolcarbonat (38%) wird erneut mit 672g (21 Mol) Methanol und dem als Destillationsrück-

stand verbliebenen Katalysator aus 1a umgesetzt, indem man das Gemisch in 30' im Autoklaven auf 150°C bringt und wieder abkühlt.

Nach Aufarbeitung wie in 1a erhält man 689g Methanol-Dimethylcarbonat-Gemisch, das 89g Dimethylcarbonat enthält, 267g Glykol-Glykolcarbonat-Gemisch mit 21g Glykolcarbonat und 0,3g Destillationsrückstand, d.h. 80% des aus 1a übernommenen Äthylenglykolcarbonates werden in diesem zweiten Durchsatz zu Dimethylcarbonat umgesetzt. Der Gesamtumsatz an Glykolcarbonat über 1a und 1b beträgt demnach etwa 94%, die Ausbeute an Dimethylcarbonat 352g d.s. 98—99% d.Th. Bei Berücksichtigung der Verluste wäre die Ausbeute praktisch quantitativ.

### Beispiel 2

Beispiel 1a wird wiederholt mit dem Unterschied, daß statt 0,2 g Thalliumcarbonat 0,2g Thalliumnitrat als Katalysator verwendet werden und die Reaktion 15' bei 180° durchgeführt wird. Nach Aufarbeitung wie in Beispiel 1 stellt man fest, daß der Umsatz an Glykolcarbonat 66% und der Destillationsrückstand 0,25g beträgt. Polyglykole werden also nicht gefunden. Die Ausbeute an Dimethylcarbonat entspricht der von Beispiel 1.

### Beispiel 3 (Vergliechsbeispiel) Ein Gemisch wie in Beispiel 1a wird in Gegenwart von 0,15g Tributylamin (der 0,2g $Tl_2CO_3$ äquivalenten Menge) als Katalysator 2h auf 150°C erhitzt. Nach Aufarbeitung wie unter 1a findet man folgendes Ergebnis: Das Reaktionsgemisch ist gelblich, der Umsatz an Glykolcarbonat beträgt 47%; davon sind ca. 3% in Polyglykole übergegangen. Das Destillat ist stickstoff- und damit katalysatorhaltig. Die Umesterungsgeschwindigkeit ist um mind. den Faktor 20 langsamer als beim $Tl_2CO_3$, die Ausbeute an Dimethylcarbonat um 3% kleiner und die Abtrennung des Katalysators schwieriger.

### Beispiel 4 (Vergleichsbeispiel)

Um mit Tributylamin die gleiche Umesterungsgeschwindigkeit wie mit $Tl_2CO_3$ zu erreichen, muß bei gleichem Ansatz wie in Vergleichsbeispiel 1 die Menge an Tributylamin auf 5g erhöht werden.

Die Aufarbeitung ergibt folgendes: Der Umsatz an Glykolcarbonat zu Dimethylcarbonat beträgt 63%. Als Destillationsrückstand bleiben 35g Polyglykole, d.h. bei gleicher Reaktionsgeschwindigkeit wirkt das Amin weit weniger selektiv als das Thalliumsalz.

### Beispiel 5

Ein Gemisch von 640g (21,5 Mol) Äthanol, 264g (3,0 Mol) Glykolcarbonat und 0,1g Thalliumhydroxid wird 2h bei 150°C gehalten und wie in Beispiel 1 aufgearbeitet. Man erhält einen Umsatz an Glykolcarbonat zu Diäthylcarbonat von 58%. Der Destillationsrückstand beträgt 0,11g, was der eingesetzten Katalysatormenge entspricht, Di- und Triglykol sind im Destillat nicht nachweisbar.

### Vergleichsbeispiel

Dieses Beispiel stellt eine Nacharbeitung des Ansatzes 2 der Tabelle der US—PS 3 642 858 dar: Ein Gemisch von 640g (13,9 Mol) Äthanol, 264g (3,0 Mol) Glykolcarbonat und 3,0g Natriumäthylat wird 3 Stunden auf 200°C erhitzt. Nach Abkühlen wird sie wie im Beispiel 1 aufgearbeitet. Man erhält eine Äthanolfraktion (687g) mit 102g Diäthylcarbonat entsprechend einem Umsatz an Glykolcarbonat zu Diäthylcarbonat von 29%, eine Glykolfraktion (133g 83 bis 102°C/400 Pa) und 59g Rückstand dickflüssigen Polyglykolen, aus denen die Glykolfraktion nur unter Zersetzungserscheinungen herausdestilliert werden kann. Der Aufarbeitungs- und Substanzverlust ($CO_2$-Abspaltung) beträgt 5 Gew.-%. Circa 30% des eingesetzten Glykolcarbonats sind in Nebenprodukte übergegangen.

### Beispiel 6

Man erhitzt 3,52kg (40 Mol) Glykolcarbonat, 300g Methanol und 0,25g Thallium-I-hexahydrobenzoat an einer 1,3m hohen Füllkörperkolonne auf 115—125°C, während gleichzeitig im Zeitraum von 20 Minuten jeweils etwa 200g Methanol über einen auf 210°C beheizten Verdampfungskolben in das Reaktionsgemisch eingeführt werden. Über Kopf der Kolonne geht ein aus Dimethylcarbonat und Methanol bestehendes Gemisch vom Siedepunkt 63,7—64°C über. Des Gehalt an Dimethylcarbonat wird anhand eines aus Gemischen bekannter Zusammensetzung erstellten Diagramms der Brechungsindices bestimmt. Nach 22 Stunden Reaktionszeit sind 10,85kg Methanol eingeführt und 10,72kg Destillat mit einem Gehalt von 1,34kg Dimethylcarbonat (10,9 Mol) übergetrieben. Der Rückstand (3,2kg) enthält nach analytischer Bestimmung des Carbonats 2,19kg (24,9 Mol) unumgesetztes Glykolcarbonat. Der Verbrauch an Glykolcarbonat beträgt 15,1 Mol. Ausbeute 99% bezogen auf umgesetztes Glykolcarbonat.

### Beispiel 7

Entsprechend Beispiel 6 werden 40 Mol Glykolcarbonat innerhalb 32 Stunden mit 12,4kg Methanol bei 120—125°C Innentemperatur in Gegenwart von 1g Thallium-I-nitrat umgesetzt. In 11,02kg abdestilliertem Azeotropgemisch sind 1,482kg = 16,5 Mol Dimethylcarbonat enthalten 1,93kg = 22 Mol Glykolcarbonat bleiben unumgesetzt. Die Ausbeute bezogen auf umgesetztes Glykolcarbonat (18 Mol) beträgt somit 92% der Theorie.

### Beispiel 8

Entsprechend Beispiel 6 werden 40 Mol Glykolcarbonat innerhalb 20 Stunden mit 11,1 kg Methanol bei 120—123°C in Gegenwart von 0,5g Thallium-I-carbonat umgesetzt. In 11,16kg Destillat sind 1,44kg = 16,3 Mol Dimethylcarbonat enthalten. 2,02kg = 22,9 Mol Glykolcarbonat bleiben unumgesetzt. Die Ausbeute bezogen auf umgesetztes Glykolcarbonat (17,1 Mol) beträgt somit 96% der Theorie.

### Beispiel 9 (Vergleichsbeispiel) Entsprechend Beispiel 7 werden 40 Mol Glykolcarbonat innerhalb 32h mit 12,0kg Methanol bei 120—123°C in Gegenwart von 1g Natriumhydroxid umgesetzt. In 10,5kg abdestilliertem Azeotropgemisch sind 1,185kg = 13,2 Mol Dimethylcarbonat enthalten. Nach $CO_2$-Bestimmung durch Verseifung sind im Rückstand 1,872kg = 21,3 Mol Glykolcarbonat unumgesetzt geblieben. Ausbeute bezogen auf umgesetztes Glykolcarbonat (18,7 Mol) 71% der Theorie.

### Beispiel 10 (Vergleichsbeispiel) Entsprechend Beispiel 7 werden 40 Mol Glykolcarbonat innerhalb 32 Stunden mit 8,5kg Methanol bei 120—124°C in Gegenwart von 1g Lithiumhydroxid umgesetzt. In 7,9kg abdestilliertem Azeotropgemisch sind 1,162kg = 13,2 Mol Dimethylcarbonat enthalten. 1,805kg = 20,5 Mol Glykolcarbonat sind im Rückstand unumgesetzt enthalten. Ausbeute bezogen auf umgesetztes Glykolcarbonat (19,5 Mol) 68% der Theorie.

### Beispiel 11 (Vergleichsbeispiel) Entsprechend Beispiel 7 werden 40 Mol Glykolcarbonat innerhalb 32 Stunden mit 9,2kg Methanol bei 120—125°C in Gegenwart von 2,5g Triäthanolamin umgesetzt. In 8,2kg Destillat sind 1,366kg = 15,1 Mol Dimethylcarbonat enthalten. Im Rückstand sind 1,88kg = 20,9 Mol Glykolcarbonat unumgesetzt. Die Ausbeute bezogen auf 19,1 Mol umgesetztes Glykolcarbonat beträgt somit 79% der Theorie.

### Beispiel 12

Ein Gemisch von 576g (18 Mol) Methanol, 88g (1 Mol) Glykolcarbonat und 0,1g Thalliumcarbonat wird unter Normaldruck zum Sieden erhitzt, während am Kopf einer 1,70m Füllkörperkolonne bei 63°C das Dimethylcarbonat/Methanol-Azeotrop abgenommen wird. Nach 45 Stunden ist die Umesterung beendet und 290g Destillat, entsprechend 85,5g Dimethylcarbonat, sind abgetrennt. Im Rückstand sind neben dem übrigen Methanol und dem gebildeten Glykol nur 0,17g Diglykol, aber keine Polyglykole nachweisbar. Ausbeute an Dimethylcarbonat 95% der Theorie.

Bei Verwendung von Triäthylamin als Katalysator werden, will man etwa die gleiche Umesterungsgeschwindigkeit erzielen, 1,5g, das 24-fache Äquivalent der obigen Katalysatormenge, benötigt. Die Umesterung ist dann in 52h beendet. An Diglykol sind 1,5g, an Triglykol 0,4g im Rückstand enthalten.

### Beispiel 13

Ein Gemisch von 661g (17,5 Mol) Methanol, 457g (4,48 Mol) Propylencarbonat und 0,1g Thalliumcarbonat wird 2 Stunden auf 150°C erhitzt. Nach Abkühlen und Aufarbeitung wie in Beispiel 2 erhält man 150g Dimethylcarbonat entsprechend einem Umsatz an Propylencarbonat von 32%. Der Rückstand beträgt 1g, die Ausbeute an Dimethylcarbonat bezogen auf umgesetztes Propylencarbonat 98,5%.

## Patentansprüche

1. Verfahren zur Herstellung von Dialkylcarbonaten durch Umsetzung von Glykolcarbonaten mit Alkoholen bei erhöhter Temperatur in Gegenwart von Katalysatoren, dadurch gekennzeichnet, daß man die Reaktion in Gegenwart von Thalliumverbindungen als Katalysatoren bei Temperaturen im Bereich von 50 bis 250°C durchführt.

2. Verfahren nach Anspruch 1, dadurch gekennzeichnet, daß man bei Temperaturen im Bereich von 60 bis 220°C arbeitet.

3. Verfahren nach Anspruch 1, dadurch gekennzeichnet, daß man 10 bis 0,0001 Gew.-% Thalliumverbindungen, bezogen auf das Reaktionsgemisch, verwendet.

4. Verfahren nach Anspruch 1, dadurch gekennzeichnet, daß man 0,001 bis 1 Gew.-% Thalliumverbindungen, bezogen auf das Reaktionsgemisch, verwendet.

5. Verfahren nach Anspruch 1, dadurch gekennzeichnet, daß man einen Überschuß von 1 bis 10 Mol Alkohol pro Mol Glykolcarbonat einsetzt.

6. Verfahren nach Ansprüchen 1 bis 5, dadurch gekennzeichnet, daß man als Thalliumverbindungen Thallium-I-oxid, Thallium-I-hydroxid, Thallium-I-carbonat, Thallium-I-acetat, Thallium-III-acetat, Thallium-I-fluorid, Thallium-I-formiat, Thallium-I-nitrat, Thallium-I-naphthenat und Thalliummethylat einsetzt.

## Claims

1. Process for the preparation of dialkyl carbonates by reacting glycol carbonates with alcohols at elevated temperatures in the presence of catalysts, characterised in that the reaction is carried out in the presence of thallium compounds as the catalysts at temperatures in the range from 50 to 250°C.

2. Process according to Claim 1, characterised in that the reaction is carried out at temperatures in the range from 60 to 220°C.

3. Process according to Claim 1, characterised in that 10 to 0.0001% by weight of thallium compounds, relative to the reaction mixture, is used.

4. Process according to Claim 1, characterised in that 0.001 to 1% by weight of thallium compounds, relative to the reaction mixture, is used.

5. Process according to Claim 1, characterised in that an excess of 1 to 10 mols of alcohol per mol of glycol carbonate is employed.

6. Process according to Claims 1 to 5, characterised in that thallium-I oxide, thallium-I hydroxide, thallium-I carbonate, thallium-I acetate, thallium-III acetate, thallium-I fluoride, thallium-I formate, thallium-I nitrate, thallium-I naphthenate and thallium methylate are employed as the thallium compounds.

**Revendications**

1. Procédé de fabrication de carbonates de dialcoyle par réaction de carbonates de glycols avec des alcools à température élevée en présence de catalyseurs, caractérisé en ce qu'on exécute la réaction en présence de composés de thallium comme catalyseurs à des températures dans l'intervalle de 50 à 250°C.

2. Procédé selon la revendication 1, caractérisé en ce qu'on opère à des températures dans l'intervalle de 60 à 220°C.

3. Procédé selon la revendication 1, caractérisé en ce qu'on utilise 10 à 0,0001% en poids de composés de thallium par rapport au mélange de réaction.

4. Procédé selon la revendication 1, caractérisé en ce qu'on utilise 0,001 à 1% en poids de composés de thallium par rapport au mélange de réaction.

5. Procédé selon la revendication 1, caractérisé en ce qu'on utilise un excès de 1 à 10 moles d'alcool par mole de carbonate de glycol.

6. Procédé selon l'une quelconque des revendications 1 à 5, caractérisé en ce qu'on utilise comme composés de thallium de l'oxyde de thallium-I, de l'hydroxyde de thallium-I, du carbonate de thallium-I, de l'acétate de thallium-I, de l'acétate de thallium-III, du fluorure de thallium-I, du formiate de thallium-I, du nitrate de thallium-I, du naphthénate de thallium-I et du méthylate de thallium.